# EUROPEAN PATENT SPECIFICATION

(11) **EP 2 101 780 B1**
(45) Date of publication and mention of the grant of the patent: **09.10.2013**
(21) Application number: 07868831.4
(22) Date of filing: 21.11.2007
(51) Int. Cl.: A61K 31/519, A61K 39/00, A61K 31/505, A61K 45/06, A61P 29/00, C07K 16/24, A61K 39/395

(54) **METHODS OF TREATING CHRONIC INFLAMMATORY DISEASES USING A GM-CSF ANTAGONIST**
VERFAHREN ZUR BEHANDLUNG CHRONISCHER ENTZÜNDUNGSKRANKHEITEN MITHILFE EINES GM-CSF-AGONISTEN
PROCÉDÉS DE TRAITEMENT DES MALADIES INFLAMMATOIRES CHRONIQUES À L'AIDE D'UN ANTAGONISTE DU GM-CSF

(30) Priority: 21.11.2006 US 860780 P; 21.02.2007 US 902742 P
(43) Date of publication of application: 23.09.2009
(60) Divisional application: 11182971.9
(73) Proprietor: Kalobios Pharmaceuticals, Inc., Palo Alto, CA 94304 (US)
(72) Inventor: BEBBINGTON, Christopher R., San Mateo, California 94402 (US); YARRANTON, Geoffrey T., Burlingame, California 94010 (US)
(74) Representative: Campbell, Patrick John Henry
(86) International application number: PCT/US2007/085402
(87) International publication number: WO 2008/064321

(56) References cited:
- KRINNER EVA-MARIA ET AL: "A human monoclonal IgG1 potently neutralizing the pro-inflammatory cytokine GM-CSF" MOLECULAR IMMUNOLOGY, ELMSFORD, NY, US, vol. 44, no. 5, 11 May 2006 (2006-05-11), pages 916-925, XP002404704 ISSN: 0161-5890
- PULJIC ET AL: "Lipopolysaccharide-induced lung inflammation is inhibited by neutralization of GM-CSF" EUROPEAN JOURNAL OF PHARMACOLOGY, AMSTERDAM, NL, vol. 557, no. 2-3, 14 November 2006 (2006-11-14), pages 230-235, XP005872830 ISSN: 0014-2999
- RIKSEN N P ET AL: "Methotrexate modulates the kinetics of adenosine in humans in vivo" ANNALS OF RHEUMATOLOGICAL DISEASES, vol. 65, 24 November 2005 (2005-11-24), pages 465-470, XP002484469
- GONG ET AL: "The nonspecific anti-inflammatory therapy with methotrexate for patients with chronic heart failure" AMERICAN HEART JOURNAL, MOSBY- YEAR BOOK INC., ST. LOUIS, MO, US, vol. 151, no. 1, 1 January 2006 (2006-01-01), pages 62-68, XP005228242 ISSN: 0002-8703
- G R BURMESTER: 'Cam-3001: A Novel Human Monoclonal Antibody against GM-CSFR-, in Subjects with Rheumatoid Arthritis (RA)-Results of a Phi Study' ARTHRITIS & RHEUMATISM, ABSTRACT SUPPLEMENT vol. 60, 16 October 2009, PHILADELPHIA, page 1926, XP055040094 DOI: 10.1002/art.26999
- ALLAN GIBOFSKY: "Combination Therapy for Rheumatoid Arthritis in the Era of Biologicals", HSS JOURNAL ; THE MUSCULOSKELETAL JOURNAL OF HOSPITAL FOR SPECIAL SURGERY, SPRINGER-VERLAG, NE, vol. 2, no. 1, 1 February 2006 (2006-02-01), pages 30-41, XP019371335, ISSN: 1556-3324, DOI: 10.1007/S11420-005-0133-Z
- R. N. MAINI ET AL: 'Anti-Cytokine Therapy for Rheumatoid Arthritis' ANNUAL REVIEW OF MEDICINE vol. 51, no. 1, 01 February 2000, pages 207 - 229, XP055012653 DOI: 10.1146/annurev.med.51.1.207 ISSN: 0066-4219
- PETER E. LIPSKY ET AL: 'Infliximab and Methotrexate in the Treatment of Rheumatoid Arthritis' NEW ENGLAND JOURNAL OF MEDICINE vol. 343, no. 22, 30 November 2000, pages 1594 - 1602, XP055012661 DOI: 10.1056/NEJM200011303432202 ISSN: 0028-4793
- E. STANLEY ET AL: 'Granulocyte/Macrophage Colony-Stimulation Factor-Deficient Mice Show no Major Perturbation of Hematopoiesis but Develop a Characteristic Pulmonary Pathology' PROCEEDINGS OF THE NATIONAL ACADEMY OF SCIENCES vol. 91, no. 12, 07 June 1994, pages 5592 - 5596, XP055012672 DOI: 10.1073/pnas.91.12.5592 ISSN: 0027-8424
- M SOSIN: 'Low dose methotrexate and bone marrow suppression.' BRIT. MED. J. vol. 326, 01 February 2003, pages 266 - 267, XP055012610 DOI: 10.1136/bmj.326.7383.266 & ANONYMOUS: "Triprim 100 mg-Tabletten", 19931006, 6 October 1993 (1993-10-06), pages 1-3, XP007919770,
- FLEISCHMANN ROY ET AL: "Anakinra: an inhibitor of IL-1 for the treatment of rheumatoid arthritis", EXPERT OPINION ON BIOLOGICAL THERAPY, INFORMA HEALTHCARE, UK, vol. 4, no. 8, 1 August 2004 (2004-08-01), pages 1333-1344, XP009154245, ISSN: 1744-7682
- ANONYMOUS: "Remicade/Infliximab (Scientific Discussion)", 20050101, 1 January 2005 (2005-01-01), pages 1-30, XP007919771,
- TARKOWSKI E ET AL.: "Local and systemic GM-CSF increase in Alzheimer's disease and vascular dementia", ACTA NEUROL SCAND, vol. 103, no. 3, March 2001 (2001-03), pages 166-174, XP007909690,

## Description

### BACKGROUND OF THE INVENTION

Rheumatoid arthritis (RA) is a chronic and typically progressive inflammatory disease that affects up to 1% of the adult population worldwide (Gabriel, Rheum Dis Clin North Am 27:269-81, 2001). Current recommendations for treatment of RA include early treatment with disease modifying anti-rheumatic drugs (DMARDs) after the diagnosis has been established. Non-steroidal anti-inflammatory drugs (NSAIDs), and until recently, COX-2 inhibitors have been widely used while waiting to confirm the diagnosis or later in the course of the disease in conjunction with DMARDs. Methotrexate is the most widely used DMARD, but other agents, including hydroxychloroquine, sulfasalazine, gold, minocycline, and leflunomide, are also prescribed. Corticosteroids may be used in combination with DMARDs, but in general, only low doses are used to minimize adverse events (O'Dell, New Engl. J. Med. 350:2591-2603, 2004).

Several new biological drugs have recently been approved for RA treatment. Etanercept (Enbrel ®), blocks Tumour Necrosis Factor alpha (TNF-α); infliximab and adalimumab (Remicade® and Humira®, respectively) block TNF-α and TNF-β; and Anakinra (Kineret®) is an inhibitor of IL-1. These agents act rapidly and have been shown to be disease modifying (slow joint/bone erosion) (Olsen & Stein, New Engl. J. Med 350:2167-2179, 2004). However, some problems remain with these therapies. Some patients do not achieve an adequate response to the TNF inhibitors. Furthermore, in some patients, the therapeutic benefit of the TNF inhibitor is lost over time. Blocking the TNF pathway has also been associated with reactivation of tuberculosis as well as increased risk of severe infections, demyelination, and lymphoma, although RA patients are at higher risk for lymphoma than the general population. Anakinra has a short half-life and must be given as a daily injection and hence, is used less frequently than the longer acting TNF inhibitors as a first line biological therapy.

Recent data on the use of rituximab (Mabthera®), a monoclonal anti-CD20 antibody, in combination with methotrexate in patients with RA has shown benefit over an extended period of time after two infusions of the antibody (Edwards et al., New Engl. J. Med 350: 2572-2581, 2004).

Methotrexate is used as a DMARD to treat RA and other inflammatory arthritic diseases and autoimmune indications, including psoriasis and systemic lupus erythemaotosus. Methotrexate is particularly effective for treating psoriatic arthritis and juvenile idiopathic arthritis. The drug is also used in chemotherapy of cancer at higher doses than are recommended for the treatment of inflammatory arthritis. When used in chemotherapy, methotrexate can cause bone-marrow suppression resulting in decreased production of all kinds of blood cells, particularly when used in combination with any of a number of other drugs including corticosteroids, non-steroidal anti-inflammatory drugs, cyclosporin, trimethoprim and certain antibiotics. Although methotrexate is generally well tolerated in the dosing regimens used for the treatment of arthritis (or psoriasis), even at these lower doses methotrexate can cause bone-marrow suppression and especially neutropenia. For example, in case reports (Sosin & Handa, Brit. Med. J. 326: 266-267, 2003) neutropenia was reported in patients treated with weekly doses of methotrexate of between 5 mg and 17.5 mg. Recommendations for methotrexate dosing in RA, such as the British Society for Rheumatology's guidelines (July 2000) are 7.5 mg Methotrexate weekly, increasing by 2.5 mg every six weeks to a maximum weekly dose of 25 mg. Thus neutropenia developed in these patients at doses significantly below the recommended maximum dose, especially with concomitant therapies.

In chemotherapy including methotrexate, GM-CSF may be prescribed to correct the low neutrophil levels in the blood and hence reduce the duration and severity of the neutropenia (Am. Soc. Clin. Onc. 2006, J. Clin. Oncol. 24: July 1st 2006). In this clinical setting, GM-CSF is used as a hematopoietic growth factor to enhance the production of granulocytes (including neutrophils) and macrophages. For example, short-term administration of GM-CSF to cancer patients can lead to a rapid increase in neutrophil counts and reduces neutropenia in patients treated with chemotherapy regime including methotrexate (Aglietta et al., Cancer 72: 2970-2973, 1993). The established efficacy of GM-CSF in treating neutropenia due to methotrexate raises concerns that antagonism of GM-CSF may have the opposite effect, i.e., GM-CSF antagonism may contribute to neutropenia, particularly in patients concomitantly or previously treated with methotrexate.

Krinner et al. (Mol Immunol 2006, 44(5): 916-25) describes a human monoclonal IgG1 that neutralizes GM-CSF. Puljic et al. (Eur J Pharmacol 2006, 557: 230-5) describes a GM-CSF neutralizing antibody in a mouse model for lung inflammation. Riksen et al. (Ann Rheum Dis 2005, 65(4): 465-70) describes the treatment of patients with arthritis with methotrexate. Gong et al. (Am Heart J 2006, 151(1): 62-8) describes the addition of methotrexate to conventional therapy for patients with chronic heart failure. Tarkowski et al. (Acta Neurol Scand 2001, 103: 166-174) describes the local and systemic increase of GM-CSF in Alzheimer's disease and vascular dementia.

Neutropenia is a significant and serious side-effect of several current therapies for inflammatory arthritis including cytokine antagonists. The IL-1 antagonist anakinra leads to an increased risk of neutropenia, both alone and particularly when used in combination with a TNF-antagonist (Fleischmann et al., Expert Opinion Biol Ther. 4:1333, 2004). Infliximab has also been associated with an increased risk of neutropenia.

There is currently a need for additional treatments of RA, particularly in patients receiving methotrexate. The current invention addresses this need.

### BRIEF SUMMARY OF THE INVENTION

The present invention provides an anti-GM-CSF antibody for use in reducing the symptoms of rheumatoid arthritis in a patient undergoing treatment with methotrexate without inducing clinically significant neutropenia in which the absolute neutrophil count is less than 0.5x10⁹/l, wherein the anti-GM-CSF antibody is a GM-CSF antagonist, wherein the methotrexate is provided in an amount of 7.5 mg to 25 mg/week, and wherein the anti-GM-CSF antibody is provided in an amount of 0.1 to 25 mg/kg. In some embodiments, the anti-GM-CSF antibody is recombinantly produced, e.g., is a recombinant monoclonal antibody. In other embodiments, the anti-GM-CSF antibody, e.g., purified anti-GM-CSF from human plasma, is purified from a natural source.

In various embodiments, the antibody can be a polyclonal antibody, a monoclonal antibody, or an antibody such as a nanobody or a camelid antibody. In some embodiments, the antibody is an antibody fragment, such as a Fab, a Fab', a F(ab')₂, a scFv, or a domain antibody (dAB). The antibody can also be modified, *e.g.,* to enhance stability. Thus, in some embodiments, the antibody is conjugated to polyethylene glycol.

In some embodiments, the antibody has a K_{D} of about 100 pM to about 10 nM, *e.g.,* from about 100 pM, about 200 pM, about 300 pM, about 400 pM, about 500 pM, about 600 pM, about 700 pM, about 800 pM, about 900 pM, or about 1 nM to about 10 nM. In further embodiments, the antibody has a K_{D} of about 1 pM to about 100 pM, *e.g.,* a K_{D} of about 1 pM, about 5 pM, about 10 pM, about 15 pM, about 20 pM, about 25 pM, about 30 pM, about 40 pM, about 50 pM, about 60 pM, about 70 pM, about 80 pM, or about 90 pM to about 100 pM. In some embodiments, the antibody has a K_{D} of from about 10 to about 30 pM.

In some embodiments, the antibody is a neutralizing antibody. In further embodiments, the antibody is a recombinant or chimeric antibody. In some embodiments, the antibody is a human antibody. In some embodiments, the antibody comprises a human variable region. In some embodiments, the antibody comprises a human light chain constant region. In some embodiments, the antibody comprises a human heavy chain constant region, such as a gamma chain.

In further embodiments, the antibody binds to the same epitope as a chimeric 19/2 antibody. The antibody can, *e.g.,* comprise the V_{H} and V_{L} regions of chimeric 19/2. The antibody can also comprise a human heavy chain constant region such as a gamma region. In some embodiments, the antibody comprises the CDR1, CDR2, and CDR3 of the V_{H} region of chimeric 19/2. In further embodiments, the antibody comprises the CDR1, CDR2, and CDR3 of the V_{L} region of chimeric 19/2. In additional embodiments, the antibody comprises the CDR1, CDR2, and CDR3 of the V_{H} and V_{L} regions of a chimeric 19/2 antibody. In some embodiments, the antibody comprises the V_{H} region CDR3 and V_{L} region CDR3 of chimeric 19/2.

In some embodiments, the antibody has a half-life of about 7 to about 25 days.

In some embodiments, the anti-GM-CSF antibody, is administered by injection or by infusion. For example, the anti-GM-CSF antibody can be administered intravenously over a period between about 15 minutes and about 2 hours.

In other embodiments, the anti-GM-CSF antibody is administered subcutaneously by bolus injection.

In further embodiments, the anti-GM-CSF antibody is administered intramuscularly.

A GM-CSF antibody can, for example, be administered at a dose between about 1 mg/kg of body weight and about 10 mg/kg of body weight.

In some embodiments, treatment with the anti-GM-CSF antibody comprises a second administration of the anti-GM-CSF antibody.

### DETAILED DESCRIPTION OF THE INVENTION

### Definitions

As used herein, "chronic inflammatory disease" refers to diseases associated with an inflammatory response of prolonged duration. In some instances, the inflammatory response can last weeks, months or even indefinitely. The extended duration of the inflammatory response is frequently provoked by a persistent stimulus to the inflammatory response. The inflammatory response causes tissue damage. Chronic inflammation can be the result of progression of acute inflammation. Chronic inflammation can also ensue after repeat episodes of acute inflammation or can develop *de novo.* A number of inflammatory illnesses have been found to be associated with persistent pathogen infection, irritant non-living foreign matter that cannot be removed by enzymatic break-down or phagocytosis, or a "normal" tissue component that is recognized as non-self (most frequently associated with auto-immune diseases). The histological appearance of chronic inflammation frequently involves a mixed inflammatory cell infiltrate which is most often associated with the presence of macrophages, lymphocytes and plasma cells with neutrophil and eosinophil polymorphs as possible minor components (neutrophil and eosinophil polymorphs are associated in greater numbers with acute inflammation). Examples of inflammatory diseases include arthritis, *e.g.,* RA, psoriatic arthritis, ankylosing spondylitis, juvenile idiopathic arthritis, and other inflammatory diseases of the joint; inflammatory bowel diseases, *e.g.,* ulcerative colitis, Crohn's disease, Barrett's syndrome, ileitis, enteritis, and gluten-sensitive enteropathy; inflammatory disorders of the respiratory system, such as asthma, adult respiratory distress syndrome, allergic rhinitis, silicosis, chronic obstructive airway disease, hypersensitivity lung diseases, bronchiectasis; inflammatory diseases of the skin, including psoriasis, scleroderma, and inflammatory dermatoses such as eczema, atopic dermatitis, urticaria, and pruritis; disorders involving inflammation of the central and peripheral nervous system, including multiple sclerosis, idiopathic demyelinating polyneuropathy, Guillain-Barre syndrome, chronic inflammatory demyelinating polyneuropathy, and neurodegenerative diseases such as Alzheimer's disease. Various other inflammatory diseases can be treated using the methods of the invention. These include systemic lupus erythematosis, immune-mediated renal disease, e.g., glomerulonephritis, and spondyloarthropathies; and diseases with an undesirable chronic inflammatory component such as systemic sclerosis, idiopathic inflammatory myopathies, Sjogren's syndrome, vasculitis, sarcoidosis, thyroiditis, gout, otitis, conjunctivitis, sinusitis, sarcoidosis, Behcet's syndrome, hepatobiliary diseases such as hepatitis, primary biliary cirrhosis, granulomatous hepatitis, and sclerosing cholangitis; inflammation and ischemic injury to the cardiovascular system such as ischemic heart disease, stroke, and atherosclerosis; and graft rejection, including allograft rejection and graft-v-host disease. Various other inflammatory diseases include tuberculosis and chronic cholecystitis. Additional chronic inflammatory diseases are described, e.g., in Harrison's Principles of Internal Medicine, 12th Edition, Wilson, et al., eds., McGraw-Hill, Inc.).

The term "rheumatoid arthritis" (RA) refers to chronic inflammatory disease that develops as an auto-immune disorder and is associated with chronic inflammation of the joints. Frequently, the inflammation spreads to tissues surrounding the joints and to other organs. Typically,RA is a progressive illness that can cause join destruction and functional disability. The joint inflammation associated with RA causes swelling, pain, stiffness, and redness in the joints. The inflammation of rheumatoid disease can also occur in tissues around the joints, such as the tendons, ligaments, and muscles. In some patients with RA, chronic inflammation leads to the destruction of the cartilage, bone and ligaments causing deformity of the joints. Damage to the joints can occur early in the disease and be progressive. Progressive damage to the joints does not necessarily correlate with the degree of pain, stiffness, or swelling present in the joints.

As used herein, "Granulocyte Macrophage-Colony Stimulating Factor" (GM-CSF) refers to a small a naturally occurring glycoprotein with internal disulfide bonds having a molecular weight of approximately 23 kDa. In humans, it is encoded by a gene located within the cytokine cluster on human chromosome 5. The sequence of the human gene and protein are known. The protein has an N-terminal signal sequence, and a C-terminal receptor binding domain (Rasko and Gough In: The Cytokine Handbook, A. Thomson, et al, Academic Press, New York (1994) pages 349-369). Its three-dimensional structure is similar to that of the interleukins, although the amino acid sequences are not similar. GM-CSF is produced in response to a number of inflammatory mediators by mesenchymal cells present in the hemopoietic environment and at peripheral sites of inflammation. GM-CSF is able to stimulate the production of neutrophilic granulocytes, macrophages, and mixed granulocyte-macrophage colonies from bone marrow cells and can stimulate the formation of eosinophil colonies from fetal liver progenitor cells. GM-CSF can also stimulate some functional activities in mature granulocytes and macrophages.

The term "granulocyte macrophage-colony stimulating factor receptor" (GM-CSFR)" refers to a membrane bound receptor expressed on cells that transduces a signal when bound to granulocyte macrophage colony-stimulating factor (GM-CSF). GM-CSFR consists of a ligand-specific low-affinity binding chain (GM-CSFR alpha) and a second chain that is required for high-affinity binding and signal transduction. This second chain is shared by the ligand-specific alpha-chains for the interleukin 3 (IL-3) and IL-5 receptors and is therefore called beta common (beta c). The cytoplasmic region of GM-CSFR alpha consists of a membrane-proximal conserved region shared by the alpha 1 and alpha 2 isoforms and a C-terminal variable region that is divergent between alpha 1 and alpha 2. The cytoplasmic region ofbeta-c contains membrane proximal serine and acidic domains that are important for the proliferative response induced by GM-CSF

The term "soluble granulocyte macrophage-colony stimulating factor receptor" (sGM-CSFR) refers to a non-membrane bound receptor that binds GM-CSF, but does not transduce a signal when bound to the ligand.

As used herein, a "peptide GM-CSF antagonist" refers to an antibody that interacts with GM-CSF to reduce or block (either partially or completely) signal transduction that would otherwise result from the binding of GM-CSF to its cognate receptor expressed on cells. Anti-GM-CSF antibodies may act by reducing the amount of GM-CSF ligand available to bind the receptor (*e.g.,* antibodies that once bound to GM-CSF increase the clearance rate of GM-CSF) or prevent the ligand from binding to its receptor by binding to GM-CSF (*e.g.,* neutralizing antibodies). An exemplary assay to detect GM-CSF antagonist activity is provided in Example 1. Typically, peptide GM-CSF antagonist, such as a neutralizing antibody, has an EC₅₀ of 10 nM or less.

A "purified" anti-GM-CSF antibody as used herein refers to an anti-GM-CSF antibody that is substantially or essentially free from components that normally accompany it as found in its native state. For example, an anti-GM-CSF antibody that is purified from blood or plasma is substantially free of other blood or plasma components such as other immunoglobulin molecules. Purity and homogeneity are typically determined using analytical chemistry techniques such as polyacrylamide gel electrophoresis or high performance liquid chromatography. A protein that is the predominant species present in a preparation is substantially purified. Typically, "purified" means that the protein is at least 85% pure, more preferably at least 95% pure, and most preferably at least 99% pure relative to the components with which the protein naturally occurs.

As used herein, an "antibody" refers to a protein functionally defined as a binding protein and structurally defined as comprising an amino acid sequence that is recognized by one of skill as being derived from the framework region of an immunoglobulin-encoding gene of an animal that produces antibodies. An antibody can consist of one or more polypeptides substantially encoded by immunoglobulin genes or fragments of immunoglobulin genes. The recognized immunoglobulin genes include the kappa, lambda, alpha, gamma, delta, epsilon and mu constant region genes, as well as myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD and IgE, respectively.

A typical immunoglobulin (antibody) structural unit is known to comprise a tetramer. Each tetramer is composed of two identical pairs of polypeptide chains, each pair having one "light" (about 25 kD) and one "heavy" chain (about 50-70 kD). The N-terminus of each chain defines a variable region of about 100 to 110 or more amino acids primarily responsible for antigen recognition. The terms variable light chain (V_{L}) and variable heavy chain (V_{H}) refer to these light and heavy chains, respectively.

The term "antibody" as used herein includes antibody fragments that retain binding specificity. For example, there are a number of well characterized antibody fragments. Thus, for example, pepsin digests an antibody below the disulfide linkages in the hinge region to produce F(ab)'₂, a dimer of Fab which itself is a light chain joined to VH-CH1 by a disulfide bond. The F(ab)'₂ may be reduced under mild conditions to break the disulfide linkage in the hinge region thereby converting the (Fab')₂ dimer into an Fab' monomer. The Fab' monomer is essentially a Fab with part of the hinge region (see, Fundamental Immunology, W.E. Paul, ed., Raven Press, N.Y. (1993), for a more detailed description of other antibody fragments). While various antibody fragments are defined in terms of the digestion of an intact antibody, one of skill will appreciate that fragments can be synthesized de novo either chemically or by utilizing recombinant DNA methodology. Thus, the term antibody, as used herein also includes antibody fragments either produced by the modification of whole antibodies or synthesized using recombinant DNA methodologies.

Antibodies include dimers such as V_{H}-V_{L} dimers, V_{H} dimers, or V_{L} dimers, including single chain antibodies (antibodies that exist as a single polypeptide chain), such as single chain Fv antibodies (sFv or scFv) in which a variable heavy and a variable light region are joined together (directly or through a peptide linker) to form a continuous polypeptide. The single chain Fv antibody is a covalently linked V_{H}-V_{L} heterodimer which may be expressed from a nucleic acid including V_{H}- and V_{L}- encoding sequences either joined directly or joined by a peptide-encoding linker (*e.g.,* Huston, et al. Proc. Nat. Acad. Sci. USA, 85:5879-5883, 1988). While the V_{H} and V_{L} are connected to each as a single polypeptide chain, the V_{H} and V_{L} domains associate non-covalently. Alternatively, the antibody can be another fragment, such as a disulfide-stabilized Fv (dsFv). Other fragments can also be generated, including using recombinant techniques. The scFv antibodies and a number of other structures converting the naturally aggregated, but chemically separated light and heavy polypeptide chains from an antibody V region into a molecule that folds into a three dimensional structure substantially similar to the structure of an antigen-binding site are known to those of skill in the art (see e.g., U.S. Patent Nos. 5,091,513, 5,132,405, and 4,956,778). In some embodiments, antibodies include those that have been displayed on phage or generated by recombinant technology using vectors where the chains are secreted as soluble proteins, *e.g.,* scFv, Fv, Fab, (Fab')₂ or generated by recombinant technology using vectors where the chains are secreted as soluble proteins. Antibodies for use in the invention can also include diantibodies and miniantibodies.

Antibodies of the invention also include heavy chain dimers, such as antibodies from camelids. Since the V_{H} region of a heavy chain dimer IgG in a camelid does not have to make hydrophobic interactions with a light chain, the region in the heavy chain that normally contacts a light chain is changed to hydrophilic amino acid residues in a camelid. V_{H} domains of heavy-chain dimer IgGs are called VHH domains. Antibodies for use in the current invention include single domain antibodies (dAbs) and nanobodies (*see, e.g.,* Cortez-Retamozo, et al., Cancer Res. 64:2853-2857, 2004).

As used herein, "V-region" refers to an antibody variable region domain comprising the segments of Framework 1, CDR1, Framework 2, CDR2, and Framework 3, including CDR3 and Framework 4, which segments are added to the V-segment as a consequence of rearrangement of the heavy chain and light chain V-region genes during B-cell differentiation. A "V-segment" as used herein refers to the region of the V-region (heavy or light chain) that is encoded by a V gene.

As used herein, "complementarity-determining region (CDR)" refers to the three hypervariable regions in each chain that interrupt the four "framework" regions established by the light and heavy chain variable regions. The CDRs are primarily responsible for binding to an epitope of an antigen. The CDRs of each chain are typically referred to as CDR1, CDR2, and CDR3, numbered sequentially starting from the N-terminus, and are also typically identified by the chain in which the particular CDR is located. Thus, for example, a V_{H} CDR3 is located in the variable domain of the heavy chain of the antibody in which it is found, whereas a V_{L} CDR1 is the CDR1 from the variable domain of the light chain of the antibody in which it is found.

The sequences of the framework regions of different light or heavy chains are relatively conserved within a species. The framework region of an antibody, that is the combined framework regions of the constituent light and heavy chains, serves to position and align the CDRs in three dimensional space.

The amino acid sequences of the CDRs and framework regions can be determined using various well known definitions in the art, *e.g.,* Kabat, Chothia, international ImMunoGeneTics database (IMGT), and AbM (*see, e.g.,* Johnson *et al., supra*; Chothia & Lesk, 1987, Canonical structures for the hypervariable regions of immunoglobulins. J. Mol. Biol. 196, 901-917; Chothia C. et al., 1989, Conformations of immunoglobulin hypervariable regions. Nature 342, 877-883; Chothia C. et al., 1992, structural repertoire of the human VH segments J. Mol. Biol. 227, 799-817; Al-Lazikani et al., J.Mol.Biol 1997, 273(4)). Definitions of antigen combining sites are also described in the following: Ruiz et al., IMGT, the international ImMunoGeneTics database. Nucleic Acids Res., 28, 219-221 (2000); and Lefranc,M.-P. IMGT, the international ImMunoGeneTics database. Nucleic Acids Res. Jan 1;29(1):207-9 (2001); MacCallum et al, Antibody-antigen interactions: Contact analysis and binding site topography, J. Mol. Biol., 262 (5), 732-745 (1996); and Martin et al, Proc. Natl Acad. Sci. USA, 86, 9268-9272 (1989); Martin, et al, Methods Enzymol., 203, 121-153, (1991); Pedersen et al, Immunomethods, 1, 126, (1992); and Rees et al, In Sternberg M.J.E. (ed.), Protein Structure Prediction. Oxford University Press, Oxford, 141-172 1996).

"Epitope" or "antigenic determinant" refers to a site on an antigen to which an antibody binds. Epitopes can be formed both from contiguous amino acids or noncontiguous amino acids juxtaposed by tertiary folding of a protein. Epitopes formed from contiguous amino acids are typically retained on exposure to denaturing solvents whereas epitopes formed by tertiary folding are typically lost on treatment with denaturing solvents. An epitope typically includes at least 3, and more usually, at least 5 or 8-10 amino acids in a unique spatial conformation. Methods of determining spatial conformation of epitopes include, for example, x-ray crystallography and 2-dimensional nuclear magnetic resonance. See, e.g., Epitope Mapping Protocols in Methods in Molecular Biology, Vol. 66, Glenn E. Morris, Ed (1996).

As used herein, "neutralizing antibody" refers to an antibody that binds to GM-CSF and prevents signaling by the GM-CSF receptor, or inhibits binding of GM-CSF to its receptor.

As used herein, "chimeric antibody" refers to an immunoglobulin molecule in which (a) the constant region, or a portion thereof, is altered, replaced or exchanged so that the antigen binding site (variable region) is linked to a constant region of a different or altered class, effector function and/or species, or an entirely different molecule that confers new properties to the chimeric antibody, e.g., an enzyme, toxin, hormone, growth factor, drug, etc.; or (b) the variable region, or a portion thereof, is altered, replaced or exchanged with a variable region, or portion thereof, having a different or altered antigen specificity; or with corresponding sequences from another species or from another antibody class or subclass.

As used herein, "humanized antibody" refers to an immunoglobulin molecule in which the CDRs of a recipient human antibody are replaced by CDRs from a donor antibody. Humanized antibodies may also comprise residues of donor origin in the framework sequences. The humanized antibody can also comprise at least a portion of a human immunoglobulin constant region.. Humanized antibodies may also comprise residues which are found neither in the recipient antibody nor in the imported CDR or framework sequences. Humanization can be performed using methods known in the art (*e.g.,* Jones et al., Nature 321:522-525; 1986; Riechmann et al., Nature 332:323-327, 1988; Verhoeyen et al., Science 239:1534-1536, 1988); Presta, Curr. Op. Struct. Biol. 2:593-596, 1992; U.S. Patent No. 4,816,567), including techniques such as "superhumanizing" antibodies (Tan et al., J. Immunol. 169: 1119, 2002) and "resurfacing" (*e.g.,* Staelens et al., Mol. Immunol. 43: 1243, 2006; and Roguska et al., Proc. Natl. Acad. Sci USA 91: 969, 1994).

A "humaneered" antibody in the context of this invention refers to an engineered human antibody having a binding specificity of a reference antibody. A "humaneered" antibody for use in this invention has an immunoglobulin molecule that contains minimal sequence derived from non-human immunoglobulin. Typically, an antibody is "humaneered" by joining a DNA sequence encoding a binding specificity determinant (BSD) from the CDR3 region of the heavy chain of the reference antibody to human V_{H} segment sequence and a light chain CDR3 BSD from the reference antibody to a human V_{L} segment sequence. A "BSD" refers to a CDR3-FR4 region, or a portion of this region that mediates binding specificity. A binding specificity determinant therefore can be a CDR3-FR4, a CDR3, a minimal essential binding specificity determinant of a CDR3 (which refers to any region smaller than the CDR3 that confers binding specificity when present in the V region of an antibody), the D segment (with regard to a heavy chain region), or other regions of CDR3-FR4 that confer the binding specificity of a reference antibody. Methods for humaneering are provided in US patent application publication no. 20050255552 and US patent application publication no. 20060134098.

The term "heterologous" when used with reference to portions of a nucleic acid indicates that the nucleic acid comprises two or more subsequences that are not normally found in the same relationship to each other in nature. For instance, the nucleic acid is typically recombinantly produced, having two or more sequences, e.g., from unrelated genes arranged to make a new functional nucleic acid. Similarly, a heterologous protein will often refer to two or more subsequences that are not found in the same relationship to each other in nature.

The term "recombinant" when used with reference, e.g., to a cell, or nucleic acid, protein, or vector, indicates that the cell, nucleic acid, protein or vector, has been modified by the introduction of a heterologous nucleic acid or protein or the alteration of a native nucleic acid or protein, or that the cell is derived from a cell so modified. Thus, e.g., recombinant cells express genes that are not found within the native (non-recombinant) form of the cell or express native genes that are otherwise abnormally expressed, under expressed or not expressed at all. By the term "recombinant nucleic acid" herein is meant nucleic acid, originally formed in vitro, in general, by the manipulation of nucleic acid, e.g., using polymerases and endonucleases, in a form not normally found in nature. In this manner, operably linkage of different sequences is achieved. Thus an isolated nucleic acid, in a linear form, or an expression vector formed in vitro by ligating DNA molecules that are not normally joined, are both considered recombinant for the purposes of this invention. It is understood that once a recombinant nucleic acid is made and reintroduced into a host cell or organism, it will replicate non-recombinantly, i.e., using the in vivo cellular machinery of the host cell rather than in vitro manipulations; however, such nucleic acids, once produced recombinantly, although subsequently replicated non-recombinantly, are still considered recombinant for the purposes of the invention. Similarly, a "recombinant protein" is a protein made using recombinant techniques, i.e., through the expression of a recombinant nucleic acid as depicted above.

The phrase "specifically (or selectively) binds" to an antibody or "specifically (or selectively) immunoreactive with," when referring to a protein or peptide, refers to a binding reaction that is determinative of the presence of the protein, in a heterogeneous population of proteins and other biologics. Thus, under designated immunoassay conditions, the specified antibodies bind to a particular protein sequence at least two times the background and more typically more than 10 to 100 times background.

Specific binding to an antibody under such conditions requires an antibody that is selected for its specificity for a particular protein. For example, polyclonal antibodies raised to a particular protein, polymorphic variants, alleles, orthologs, and conservatively modified variants, or splice variants, or portions thereof, can be selected to obtain only those polyclonal antibodies that are specifically immunoreactive with GM-CSF protein and not with other proteins. This selection may be achieved by subtracting out antibodies that cross-react with other molecules.

As used herein, a "therapeutic agent for a chronic inflammatory disease" refers to an agent that when administered to a patient suffering from a chronic inflammatory disease, in a therapeutically effective dose, will cure, or at least partially arrest the symptoms of the disease and complications associated with the disease.

### I. INTRODUCTION

The invention relates to administering an anti-GM-CSF antibody for the treatment of patients diagnosed with rheumatoid arthritis (RA). The patient is undergoing treatment with methotrexate. The anti-GM-CSF antibody and methotrexate are administered in an amount that does not induce clinically significant neutropenia in which the absolute neutrophil count is less than 0.5x10⁹/l. Anti-GM-CSF antibodies prevent signaling that normally results from the binding of GM-CSF to its cognate receptor.

Anti-GM-CSF antibodies suitable for use with the present invention may be monoclonal, polyclonal, chimeric, humanized, humaneered, or human.

### II. Patients

Typical patients to be treated with the anti-GM-CSF antibodies are those having RA who are also undergoing treatment with methotrexate and who have not developed neutropenia. Patients are treated with methotrexate according to established clinical guidelines and are treated with weekly doses of methotrexate in the range of 7.5 to 25 mg of methotrexate per week.

Methotrexate is structurally similar to folate and can bind to the active sites of a number of enzymes that normally use folate as a coenzyme for the biosynthesis of the purine and pyrimidine nucleotide precursors of DNA and for the interconversion of amino acids during protein biosynthesis. Methotrexate competes with the folate cofactor for enzyme binding sites, thereby inhibiting enzyme activity.

In certain instances, methotrexate can be used in a combination therapy with one or more methotrexate analogs and/or other anti-folate compounds and an anti-GM-CSF antibody. The methotrexate is administered in an amount that does not produce neutropenia. The amount is from 7.5, 10, 12.5, 15, 20 to 25 mg per week. The amount of methotrexate administered as an anti-inflammatory agent is often an amount that is two to three log orders lower than amounts of methotrexate used in treating cancer.

Methods well known in the art can be used to determine if a patient is neutropenic. The absolute neutrophil count (ANC) is used to determine if the patient is neutropenic. Patients are not considered neutropenic if the neutrophil and white blood cell counts (WBCC) are within the normal range. The normal range for neutrophils is understood to be represented by a count of greater than 1x10⁹/l; the normal range for white blood cells is understood to be represented by a count greater than 3.5x10⁹/l. Neutropenia is considered clinically significant if the ANC is less than 0.5x10⁹/l. No clinically significant neutropenia is induced in patients treated using the anti-GM-CSF antibody of the present invention. In any event, the treatment causes no detectable neutropenia.

In some embodiments, a patient that has active RA is treated with the anti-GM-CSF antibody of the invention. The response of a RA patient to a therapy and/or disease progression can be evaluated by monitoring any of the clinical parameters associated with RA. Typically, a patient that exhibits a therapeutic response to treatment is determined by a number of parameters, including pharmacological parameters. For example, American College for Rheumatology (ACR) scoring for RA (ACR 20, ACR 50 and ACR 70) can be employed. ACR composite end-points for RA include: morning stiffness, tender joint count, swollen joint count, patient pain assessment, patient global assessment, physician global assessment; erythrocyte sedimentation rate (ESR), C-reactive protein (CRP) levels in plasma, and measure of rheumatoid factor. Other pharmacodynamic markers that can be used to evaluate patient response include evaluation of neopterin levels in blood or in urine, evaluation of levels of pro-inflammatory cytokines systemically (in the blood) or locally (e.g., at a localized site of inflammation such as a joint). Pro inflammatory cytokines are well known in the art. Examples of pro-inflammatory cytokines that can be used to evaluate patient response to the therapy of this invention include, but are not limited to, TNF-α, GM-CSF, Interleukin-1, Interleukin-6, and Interleukins-8 and -17.

Administration of anti-GM-CSF antibodies with methotrexate at least partially arrests disease progression or reduces symptoms of the disease symptoms (as assessed by parameters such as the exemplary parameters noted above). Thus, administration of an anti-GM-CSF antibody and methotrexate can reduce the progression of joint erosion. Progression of joint erosion can be assessed using known techniques such as autoradiography to evaluate bone and cartilage in joints.

### III. GM-CSF antagonists

As noted above, the invention provides anti-GM-CSF antibodies for treating RA, by administering the anti-GM-CSF antibody and methotrexate to a patient suffering from the disease. Anti-GM-CSF antibodies suitable for use in the invention selectively interfere with the induction of signaling by the GM-CSF receptor by causing a reduction in the binding of GM-CSF to the receptor.

General molecular biology methods, including expression methods, can be found, *e.g.,* in instruction manuals, such as, Sambrook and Russell (2001) Molecular Cloning: A laboratory manual 3rd ed. Cold Spring Harbor Laboratory Press; Current Protocols in Molecular Biology (2006) John Wiley and Sons ISBN: 0-471-50338-X.

A variety of prokaryotic and/or eukaryotic based protein expression systems may be employed to produce a GM-CSF antibody. Many such systems are widely available from commercial suppliers. These include both prokaryotic and eukaryotic expression systems.

### GM-CSF Antibodies

The GM-CSF antagonist is an antibody that binds GM-CSF. The antibodies can be raised against GM-CSF proteins, or fragments, or produced recombinantly. Antibodies to GM-CSF of the invention can be neutralizing or can be non-neutralizing antibodies that bind GM-CSF and increase the rate of *in vivo* clearance of GM-CSF such that the GM-CSF level in the circulation is reduced. Often, the GM-CSF antibody is a neutralizing antibody.

Methods of preparing polyclonal antibodies are known to the skilled artisan (e.g., Harlow & Lane, Antibodies, A Laboratory manual (1988); Methods in Immunology). Polyclonal antibodies can be raised in a mammal by one or more injections of an immunizing agent and, if desired, an adjuvant. The immunizing agent includes a GM-CSF protein, *e.g.,* a human GM-CSF protein, or fragment thereof.

In some embodiment, a GM-CSF antibody of the invention is purified from human plasma. In such embodiments, the GM-CSF antibody is typically a polyclonal antibody that is isolated from other antibodies present in human plasma. Such an isolation procedure can be performed, *e.g.,* using known techniques, such as affinity chromatography.

In some embodiments, the anti-GM-CSF antibody is a monoclonal antibody. Monoclonal antibodies may be prepared using hybridoma methods, such as those described by Kohler & Milstein, Nature 256:495 (1975). In a hybridoma method, a mouse, hamster, or other appropriate host animal, is typically immunized with an immunizing agent, such as human GM-CSF, to elicit lymphocytes that produce or are capable of producing antibodies that will specifically bind to the immunizing agent. Alternatively, the lymphocytes may be immunized *in vitro.* The immunizing agent preferably includes human GM-CSF protein, fragments thereof, or fusion protein thereof.

Human monoclonal antibodies can be produced using various techniques known in the art, including phage display libraries (Hoogenboom & Winter, J. Mol. Biol. 227:381 (1991); Marks et al., J. Mol. Biol. 222:581 (1991)). The techniques of Cole *et al.* and Boerner *et al.* are also available for the preparation of human monoclonal antibodies (Cole et al., Monoclonal Antibodies and Cancer Therapy, p. 77 (1985) and Boerner et al., J. Immunol. 147(1):86-95 (1991)). Similarly, human antibodies can be made by introducing of human immunoglobulin loci into transgenic animals, *e.g.,* mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, *e.g.,* in U.S. Patent Nos. 5,545,807; 5,545,806; 5,569,825; 5,625,126; 5,633,425; 5,661,016, and in the following scientific publications: Marks et al., Bio/Technology 10:779-783 (1992); Lonberg et al., Nature 368:856-859 (1994); Morrison, Nature 368:812-13 (1994); Fishwild et al., Nature Biotechnology 14:845-51 (1996); Neuberger, Nature Biotechnology 14:826 (1996); Lonberg & Huszar, Intern. Rev. Immunol. 13:65-93 (1995).

In some embodiments the anti-GM-CSF antibodies are chimeric or humanized monoclonal antibodies. As noted *supra,* humanized forms of antibodies are chimeric immunoglobulins in which residues from a complementary determining region (CDR) of human antibody are replaced by residues from a CDR of a non-human species such as mouse, rat or rabbit having the desired specificity, affinity and capacity.

An antibody that is employed in the invention can be in any format. For example, in some embodiments, the antibody can be a complete antibody including a constant region, *e.g.,* a human constant region, or can be a fragment or derivative of a complete antibody, e.g., an Fd, a Fab, Fab', F(ab')₂, a scFv, an Fv fragment, or a single domain antibody, such as a nanobody or a camelid antibody. Such antibodies may additionally be recombinantly engineered by methods well known to persons of skill in the art. As noted above, such antibodies can be produced using known techniques.

In some embodiments of the invention, the antibody is additionally engineered to reduce immunogenicity, *e.g.,* so that the antibody is suitable for repeat administration. Methods for generating antibodies with reduced immunogenicity include humanization/humaneering procedures and modification techniques such as de-immunization, in which an antibody is further engineered, *e.g.,* in one or more framework regions, to remove T cell epitopes.

In some embodiments, the antibody is a humaneered antibody. A humaneered antibody is an engineered human antibody having a binding specificity of a reference antibody, obtained by joining a DNA sequence encoding a binding specificity determinant (BSD) from the CDR3 region of the heavy chain of the reference antibody to human VH segment sequence and a light chain CDR3 BSD from the reference antibody to a human VL segment sequence. Methods for humaneering are provided in US patent application publication no. 20050255552 and US patent application publication no. 20060134098.

An antibody can further be de-immunized to remove one or more predicted T-cell epitopes from the V-region of an antibody. Such procedures are described, for example, in WO 00/34317.

In some embodiments, the variable region is comprised of human V-gene sequences. For example, a variable region sequence can have at least 80% identity, or at least 85% identity, at least 90% identity, at least 95% identity, at least 96% identity, at least 97% identity, at least 98% identity, or at least 99% identity, or greater, with a human germline V-gene sequence.

An antibody of the invention can include a human constant region. The constant region of the light chain may be a human kappa or lambda constant region. The heavy chain constant region is often a gamma chain constant region, for example, a gamma-1, gamma-2, gamma-3, or gamma-4 constant region.

In some embodiments, *e.g.,* where the antibody is a fragment, the antibody can be conjugated to another molecule, *e.g.,* to provide an extended half-life *in vivo* such as a polyethylene glycol (pegylation) or serum albumin. Examples of PEGylation of antibody fragments are provided in Knight et al (2004) Platelets 15: 409 (for abciximab); Pedley et al (1994) Br. J. Cancer 70: 1126 (for an anti-CEA antibody) Chapman et al (1999) Nature Biotech. 17 : 780.

### Antibody Specificity

An antibody of the invention binds to GM-CSF. Any number of techniques can be used to determine antibody binding specificity. *See, e.g.,* Harlow & Lane, Antibodies, A Laboratory Manual (1988) for a description of immunoassay formats and conditions that can be used to determine specific immunoreactivity of an antibody.

An exemplary antibody suitable for use with the present invention is c19/2. In some embodiments, a monoclonal antibody that competes for binding to the same epitope as c19/2, or that binds the same epitope as c19/2, is used. The ability of a particular antibody to recognize the same epitope as another antibody is typically determined by the ability of the first antibody to competitively inhibit binding of the second antibody to the antigen. Any of a number of competitive binding assays can be used to measure competition between two antibodies to the same antigen. For example, a sandwich ELISA assay can be used for this purpose. This is carried out by using a capture antibody to coat the surface of a well. A subsaturating concentration of tagged-antigen is then added to the capture surface. This protein will be bound to the antibody through a specific antibody: epitope interaction. After washing a second antibody, which has been covalently linked to a detectable moiety (e.g., HRP, with the labeled antibody being defined as the detection antibody) is added to the ELISA. If this antibody recognizes the same epitope as the capture antibody it will be unable to bind to the target protein as that particular epitope will no longer be available for binding. If however this second antibody recognizes a different epitope on the target protein it will be able to bind and this binding can be detected by quantifying the level of activity (and hence antibody bound) using a relevant substrate. The background is defined by using a single antibody as both capture and detection antibody, whereas the maximal signal can be established by capturing with an antigen specific antibody and detecting with an antibody to the tag on the antigen. By using the background and maximal signals as references, antibodies can be assessed in a pair-wise manner to determine epitope specificity.

A first antibody is considered to competitively inhibit binding of a second antibody, if binding of the second antibody to the antigen is reduced by at least 30%, usually at least about 40%, 50%, 60% or 75%, and often by at least about 90%, in the presence of the first antibody using any of the assays described above.

### Epitope Mapping

In some embodiments of the invention, an antibody is employed that binds to the same epitope as a known antibody, *e.g.,* c19/2. Method of mapping epitopes are well known in the art. For example, one approach to the localization of functionally active regions of human granulocyte-macrophage colony-stimulating factor (hGM-CSF) is to map the epitopes recognized by neutralizing anti-hGM-CSF monoclonal antibodies. For example, the epitope to which c19/2 (which has the same variable regions as the neutralizing antibody LMM102) binds has been defined using proteolytic fragments obtained by enzymic digestion of bacterially synthesized hGM-CSF (Dempsey, et al., Hybridoma 9:545-558, 1990). RP-HPLC fractionation of a tryptic digest resulted in the identification of an immunoreactive "tryptic core" peptide containing 66 amino acids (52% of the protein). Further digestion of this "tryptic core" with S. aureus V8 protease produced a unique immunoreactive hGM-CSF product comprising two peptides, residues 86-93 and 112-127, linked by a disulfide bond between residues 88 and 121. The individual peptides, were not recognized by the antibody.

### Determining Binding Affinity

In some embodiments, the antibodies of the present invention have a high affinity binding for human GM-CSF. High affinity binding between an antibody and an antigen exists if the dissociation constant (K_{D}) of the antibody is < 1 nM, and preferably < 100 pM. A variety of methods can be used to determine the binding affinity of an antibody for its target antigen such as surface plasmon resonance assays, saturation assays, or immunoassays such as ELISA or RIA, as are well known to persons of skill in the art. An exemplary method for determining binding affinity is by surface plasmon resonance analysis on a BIAcore™ 2000 instrument (Biacore AB, Freiburg, Germany) using CM5 sensor chips, as described by Krinner et al., (2007) Mol. Immunol. Feb;44(5):916-25. (Epub 2006 May 11)).

### Cell Proliferation Assay for Identifying Neutralizing Antibodies

In some embodiments, the anti-GM-CSF antibodies are neutralizing antibodies, which bind in a manner that interferes with the binding of GM- CSF. Neutralizing antibodies and other GM-CSF antagonists may be identified using any number of assays that assess GM-CSF function. For example, cell-based assays for GM-CSF receptor signaling, such as assays which determine the rate of proliferation of a GM-CSF-dependent cell line in response to a limiting amount of GM-CSF, are conveniently used. The human TF-1 cell line is suitable for use in such an assay. See, Krinner et al., (2007) Mol. Immunol. In some embodiments, the neutralizing antibodies of the invention inhibit GM-CSF-stimulated TF-1 cell proliferation by at least 50% when a GM-CSF concentration is used which stimulates 90% maximal TF-1 cell proliferation. In other embodiments, the neutralizing antibodies inhibit GM-CSF stimulated proliferation by at least 90%. Thus, typically, a neutralizing antibody of the invention, has an EC₅₀ of less than 10 nM (*e.g.,* Table 1). Additional assays suitable for use in identifying neutralizing antibodies suitable for use with the present invention will be well known to persons of skill in the art.

### Exemplay Antibodies

Antibodies for use in the invention are known in the art and can be produced using routine techniques. Exemplary antibodies are described. It is understood that the exemplary antibodies can be engineered in accordance with the procedures known in the art and summarized herein to produce antibody fragments, chimeras, and the like by either chemical or recombinant technology.

An exemplary chimeric antibody suitable for use as a GM-CSF antagonist is c19/2. The c/19/2 antibody binds GM-CSF with a monovalent binding affinity of about 10pM as determined by surface plasmon resonance analysis. SEQ ID NOs 1 and 2 show the heavy and light chain variable region sequence of c19/2 (e.g., WO03/068920). The CDRs, as defined according to Kabat, are:

| | |
|---|---|
| CDRH1 | DYNIH |
| CDRH2 | YIAPYSGGTGYNQEFKN |
| CDRH3 | RDRFPYYFDY |
| CDRL1 | KASQNVGSNVA |
| CDRL2 | SASYRSG |
| CDRL3 | QQFNRSPLT. |

The CDRs can also be determined using other well known definitions in the art, e.g., Chothia, international ImMunoGeneTics database (IMGT), and AbM.

In some embodiments, an antibody used in the invention competes for binding to, or binds to, the same epitope as c19/2. The GM-CSF epitope recognized by c19/2 has been identified as a product that has two peptides, residues 86-93 and residues 112-127, linked by a disulfide bond between residues 88 and 121. The c19/2 antibody inhibits the GM-CSF-dependent proliferation of a human TF-1 leukemia cell line with an EC₅₀ of 30 pM when the cells are stimulated with 0.5 ng/ml GM-CSF. In some embodiments, the antibody used in the invention binds to the same epitope as c19/2.

An antibody for administration, such as c19/2, can be additionally humaneered. For example, the c19/2 antibody can be further engineered to contain human V gene segments.

Another exemplary neutralizing anti-GM-CSF antibody is the E10 antibody described in Li et al., (2006) PNAS 103(10):3557-3562. E10 is an IgG class antibody that has an 870 pM binding affinity for GM-CSF. The antibody is specific for binding to human GM-CSF as shown in an ELISA assay, and shows strong neutralizing activity as assessed with a TF1 cell proliferation assay.

An additional exemplary neutralizing anti-GM-CSF antibody is the MT203 antibody described by Krinner et al., (Mol Immunol. 44:916-25, 2007; Epub 2006 May 112006). MT203 is an IgG1 class antibody that binds GM-CSF with picomolar affinity. The antibody shows potent inhibitory activity as assessed by TF-1 cell proliferation assay and its ability to block IL-8 production in U937 cells.

Additional antibodies suitable for use with the present invention will be known to persons of skill in the art.

### III. Therapeutic Administration

The invention typically provides for administering methotrexate and an anti-GM-CSF antibody as a pharmaceutical composition to a patient having RA in a therapeutically effective amount using a dosing regimen suitable for treatment of the disease.

Patients suffering from RA are treated with methotrexate at a weekly dose of up to about 25 mg and an antibody specific for GM-CSF, at a dose that does not induce clinically significant neutropenia and leads to an improvement in one or more markers of inflammation. In some embodiments, the patient response to treatment is determined by showing a significant reduction in the erythrocyte sedimentation rate (ESR) to within the normal range. The normal ESR range is age and gender dependent. For men, normal ESR can be calculated according to the following formula: 0.5x (age in years). For women, normal ESR can be calculated according to the following formula: 0.5 x (age in years +10) (Wallach J. Interpretation of Laboratory Tests, 6th Edition. Little Brown and Company. 1996).

The composition can be formulated for use in a variety of drug delivery systems. One or more physiologically acceptable excipients or carriers can also be included in the compositions for proper formulation. Suitable formulations for use in the present invention are found in Remington's Pharmaceutical Sciences, Mack Publishing Company, Philadelphia, PA, 17th ed. (1985). For a brief review of methods for drug delivery, see, Langer, Science 249: 1527-1533 (1990).

The anti-GM-CSF antibody of the invention is provided in a solution suitable for injection into the patient such as a sterile isotonic aqueous solution for injection. The anti-GM-CSF antibody is dissolved or suspended at a suitable concentration in an acceptable carrier. In some embodiments the carrier is aqueous, e.g., water, saline, phosphate buffered saline, and the like. The compositions may contain auxillary pharmaceutical substances as required to approximate physiological conditions, such as pH adjusting and buffering agents, tonicity adjusting agents, and the like.

Pharmaceutical compositions are administered to a patient suffering from RA in an amount sufficient to cure or at least partially arrest the disease or symptoms of the disease and its complications. An amount adequate to accomplish this is defined as a "therapeutically effective dose." A therapeutically effective dose is determined by monitoring a patient's response to therapy. Typical benchmarks indicative of a therapeutically effective dose are known in the art. For RA, benchmarks include plasma levels of CRP, ESR, blood or urine levels ofneopterin, levels of pro-inflammatory cytokines (*e.g.,* TNF-α, GM-CSF, Interleukin-1, Interleukin-6 and Interleukins 8 and 17) or changes in the levels of other pharmacodynamic markers. Other criteria for assessing a therapeutic response can also be used, *e.g.,* by evaluating the number and/or severity of tenderness and swelling of the joints, pain levels, and the like.

Amounts that are administered that are effective will depend upon the severity of the disease and the general state of the patient's health, including other factors such as age, weight, gender, administration route, etc. Single or multiple administrations of the antibody may be administered depending on the dosage and frequency as required and tolerated by the patient. In any event, the methods provide a sufficient quantity of anti-GM-CSF antibody in conjunction with methotrexate to effectively treat the patient.

In another embodiment of the invention, the anti-GM-CSF antibody used to treat RA is provided in combination therapy with methotrexate and one or more additional agents, *e.g.,* a nonsteroidal anti-inflammatory agent. Thus, patients may receive additional therapies in order to treat their disease. Such therapies include, but are not limited to, hydroxychloroquinone, sulfasalazine, gold, minocycline, leflunomide, corticosteroids, TNF-antagonists (e.g., etanercept, infliximab or adalimumab), IL-1 antagonists (e.g., as anakinra) or anti-CD20 antibodies (e.g., rituximab). Patients can receive one or more of these additional therapeutic agents as concomitant therapy. Alternatively, patients may be treated sequentially with additional therapeutic agents.

### A. Administration

The invention provides an anti-GM-CSF antibody for the treatment of patients with RA by administering the antibody in combination with methotrexate. In some embodiments, the anti-GM-CSF antibody is administered by injection or infusion through any suitable route including but not limited to intravenous, sub-cutaneous, intramuscular or intraperitoneal routes. In an exemplary embodiment, the anti-GM-CSF antibody is diluted in a physiological saline solution for injection prior to administration to the patient. Such an antibody is administered, for example, by intravenous infusion over a period of between 15 minutes and 2 hours. In still other embodiments, the administration procedure is via sub-cutaneous or intramuscular injection.

The anti-GM-CSF antibody is administered while the patient is being treated with methotrexate. In the context of this invention a patient "being treated with methotrexate" or "undergoing treatment with methotrexate" means a patient has been prescribed methotrexate and is therefore receiving, or has recently received, a dose of methotrexate. Typically, methotrexate is taken once a week. Thus, for example, an anti-GM-CSF antibody can be administered at any time period during the week between doses. In some embodiments, the anti-GM-CSF antibody can be administered after a patient has received a methotrexate dose, but has not yet taken the next dose, e.g., over a week after the last dose of methotrexate. Such a patient is still considered to be undergoing treatment with methotrexate if the methotrexate therapy is still being prescribed for the patient.

### B. Dosing

The dose of anti-GM-CSF antibody is chosen in order to provide effective therapy for a patient that has RA. The dose is in the range of about 0.1 mg/kg body weight to about 25 mg/kg body weight or in the range about 1 mg to about 2 g per patient. The dose is often in the range of about 1 to about 10 mg/kg or approximately about 50 mg to about 1000 mg / patient. The dose may be repeated at an appropriate frequency which may be in the range once per day to once every three months, depending on the pharmacokinetics of the antagonists (e.g. half-life of the antibody in the circulation) and the pharmacodynamic response (e.g. the duration of the therapeutic effect of the antibody). In some embodiments, the *in vivo* half-life of between about 7 and about 25 days and antibody dosing is repeated between once per week and once every 3 months. In other embodiments, the antibody is administered approximately once per month.

Treatment protocols and doses for administering methotrexate are known in the art. For example, recommendations for methotrexate dosing in RA, such as the British Society for Rheumatology's guidelines (July 2000) are 7.5 mg Methotrexate weekly, increasing by 2.5 mg every six weeks to a maximum weekly dose of 25 mg.

The methotrexate and anti-GM-CSF antibody are administered in a range that does not induce detectable neutropenia. For example, patients receive methotrexate at a dose of up to about 25 mg/week and from about 0.2 to about 10 mg/kg of anti-GM-CSF antibody.

### EXAMPLES

### Example 1 - Exemplary humaneered antibodies to GM-CSF

A panel of humaneered Fab' molecules with the specificity of c19/2 were generated from epitope-focused human V-segment libraries as described in US patent application 20060134098.

Fab' fragments were expressed from *E*. *coli.* Cells were grown in 2xYT medium to an OD600 of 0.6. Expression was induced using IPTG for 3 hours at 33°C. Assembled Fab' was obtained from periplasmic fractions and purified by affinity chromatography using Streptococcal Protein G (HiTrap Protein G HP columns; GE Healthcare) according to standard methods. Fab's were eluted in pH 2.0 buffer, immediately adjusted to pH 7.0 and dialyzed against PBS pH7.4.

Binding kinetics were analyzed by Biacore 3000 surface plasmon resonance (SPR). Recombinant human GM-CSF antigen was biotinylated and immobilized on a streptavidin CM5 sensor chip. Fab samples were diluted to a starting concentration of 3 nM and run in a 3 fold dilution series. Assays were run in 10 mM HEPES, 150 mM NaCl, 0.1 mg/mL BSA and 0.005% p20 at pH 7.4 and 37°C. Each concentration was tested twice. Fab' binding assays were run on two antigen density surfaces providing duplicate data sets. The mean affinity (K_{D}) for each of 6 humaneered anti-GM-CSF Fab clones, calculated using a 1:1 Langmuir binding model, is shown in Table 1.

Fabs were tested for GM-CSF neutralization using a TF-1 cell proliferation assay. GM-CSF-dependent proliferation of human TF-1 cells was measured after incubation for 4 days with 0.5 ng/ml GM-CSF using a MTS assay (Cell titer 96, Promega) to determine viable cells. All Fabs inhibited cell proliferation in this assay indicating that these are neutralizing antibodies. There is a good correlation between relative affinities of the anti-GM-CSF Fabs and EC₅₀ in the cell-based assay. Anti-GM-CSF antibodies with monovalent affinities in the range 18 pM - 104 pM demonstrate effective neutralization of GM-CSF in the cell-based assay.

**Table 1: Affinity of anti-GM-CSF Fabs determined by surface plasmon resonance analysis in comparison with activity (EC₅₀) in a GM-CSF dependent TF-1 cell proliferation assay**

| **Fab** | **Monovalent binding affinity determined by SPR (pM)** | **EC₅₀(pM) in TF-1 cell proliferation assay** |
|---|---|---|
| 94 | 18 | 165 |
| 104 | 19 | 239 |
| 77 | 29 | 404 |
| 92 | 58 | 539 |
| 42 | 104 | 3200 |
| 44 | 81 | 7000 |

### Example 2 - Exemplary clinical protocol for delivery of anti-GM-CSF antibody

An anti-GM-CSF antibody is stored at 10 mg/ml in sterile isotonic aqueous saline solution for injection at 4°C and is diluted in either 100 ml or 200 ml 0.9% sodium chloride for injection prior to administration to the patient. The antibody is administered to a patient having RA by intravenous infusion over the course of 1 hour at a dose of between 0.2 and 10 mg/kg.

Patients for inclusion in this treatment protocol are chosen based on the following criteria: patients show signs of active RA, patients are currently receiving treatment with methotrexate wherein patients have been receiving stable doses of DMARDs for at least 6 weeks. Furthermore, patients included in this study exhibit the following symptoms: swollen joint count of at least 6 (using 66 joint count), tender joint count of at least 6 (using 68 joint count). At least two of the following criteria are also included in the inclusion criteria: ESR≥20 mm/hr, CRP≥15mg/l, early morning stiffness of ≥ 45 minutes.

Patients receive either placebo (0.9% sodium chloride for injection) or anti-GM-CSF antibody by intravenous infusion on Day 1 at one of the following doses: 0.2 mg/kg, 1.0 mg/kg, 5.0 mg/kg or 10mg/kg. Patients are monitored for 29 days. All patients continue to receive DMARDs, methotrexate at a dose of up to 25 mg/week, prednisolone up to 10 mg/day, and NSAIDs as clinically appropriate along with medication for any other medical conditions. Throughout the duration of the study the following tests are performed as a study safety assessment: physical examination, vital signs measurement, 12-lead electrocardiogram (ECG), laboratory tests including hematology, biochemistry and urinalysis, pulmonary function tests and incontinence and intensity of adverse events (AEs).

Efficacy of treatment is assessed in two stages. The primary assessment involves an ACR 20 response at any time prior to or at Day 29 of treatment. The secondary assessment includes measuring time to ACR20, proportion of patients who achieve an ACR 50 and 70 response and ESR and CRP measured at Days 8, 15 and 29.

Adverse events, serious adverse events and laboratory abnormalities are tabulated by treatment group and compared to those of the pooled placebo group. The efficacy of the anti-GM-CSF antibody is analyzed by calculating the ACR 20/50/70 responses for intention to treat using a closed testing procedure. The pooled active groups are compared with the patients treated with placebo.

### Example 3 - Treatment of a patient with methotrexate and anti-GM-CSF antibody

A patient that has active RA was treated with methotrexate and an anti-GM-CSF antibody according to the clinical protocol described in Example 2. The patient received 0.2 mg/kg anti-GM-CSF antibody. The patient was also undergoing treatment with 25 mg/week methotrexate.

Blood cell counts were determined by standard methods and included determination of the numbers of: hemoglobin (HGB); total white blood cells (WBCC); platelets (PLT); neutrophils (Neut; also called Absolute Neutrophil Count ANC); lymphocytes (LYMPH); monocytes (MONO); eosinophils (EOSIN); basophils (BASO), hematocrit (HCT). In addition, erythrocyte sedimentation rate (ESR), C-reactive protein (CRP) were determined. The blood cell counts ESR and CRP before and treatment and up to two weeks after treatment are shown in Table 2. As can be seen, after two weeks, the ESR dropped from an abnormal value of 40 to 18, which is within the normal range for an individual of the same sex and age as the treated patient, while the neutrophil count remained unchanged. Accordingly, a combination therapy comprising methotrexate treatment and treatment with an anti-GM-CSF antagonist, in this case an anti-GM-CSF antibody, provided a therapeutic benefit for the treatment of rheumatoid arthritis.

The "1" day in Table 2 indicates when anti-GM-CSF antagonist was administered. The patient had previously been treated with methotrexate and continued receiving methotrexate treatment with the anti-GM-CSF antagonist treatment.

**Table 2. Blood counts and ESR from a patient treated with weekly doses of methotrexate and administered a single dose of anti-GM-CSF antibody on Day 1. The numbers of the various cells (platelets, neutrophils, lymphocytes, etc.) are x 10⁹/L. The ESR is expressed in mm/hr.**

| Days post treatment | HGB | WBCC | PLT | NEUT | LYMPH | HCT | MONO | EOSIN | BASE | ESR |
|---|---|---|---|---|---|---|---|---|---|---|
| -2 | 133 | 5.0 | 202 | 3.04 | 1.26 | 0.4 | 0.52 | 0.16 | 0.02 | 40 |
| 1 | 127 | 4.4 | 208 | 2.64 | 1.23 | 0.38 | 0.36 | 0.15 | 0.03 | |
| 8 | 129 | 5.4 | 189 | 3.01 | 1.41 | 0.39 | 0.76 | 0.17 | 0.04 | 23 |
| 15 | 131 | 4.8 | 193 | 2.76 | 1.39 | 0.39 | 0.46 | 0.15 | 0.04 | 18 |
| 28 | 130 | 5.0 | 180 | 2.79 | 1.71 | 0.4 | 0.25 | 0.21 | 0.04 | 20 |

The above examples are provided by way of illustration only and not by way of limitation. Those of skill in the art will readily recognize a variety of noncritical parameters that could be changed or modified to yield essentially similar results.

### Exemplary sequences

SEQ ID NO 1: amino acid sequence for murine 19/2 heavy chain variable region 
Met Glu Leu Ile Met Leu Phe Leu Leu Ser Gly Thr Ala Gly Val His

Ser Glu Val Gln Leu Gln Gln Ser Gly Pro Glu Leu Val Lys Pro Gly

Ala Ser Val Lys Ile Ser Cys Lys Ala Ser Gly Tyr Thr Phe Thr Asp

Tyr Asn Ile His Trp Val Lys Gln Ser His Gly Lys Ser Leu Asp Trp

Ile Gly Tyr Ile Ala Pro Tyr Ser Gly Gly Thr Gly Tyr Asn Gln Glu

Phe Lys Asn Arg Ala Thr Leu Thr Val Asp Lys Ser Ser Ser Thr Ala

Tyr Met Glu Leu Arg Ser Leu Thr Ser Asp Asp Ser Ala Val Tyr Tyr

Cys Ala Arg Arg Asp Arg Phe Pro Tyr Tyr Phe Asp Tyr Trp Gly Gln Gly

Thr Thr Leu Arg Val Ser Ser Val Ser Gly Ser
SEQ ID NO 2: amino acid sequence for murine 19/2 light chain variable region

Met Gly Phe Lys Met Glu Ser Gln Ile Gln Val Phe Val Tyr Met Leu

Leu Trp Leu Ser Gly Val Asp Gly Asp Ile Val Met Ile Gln Ser Gln

Lys Phe Val Ser Thr Ser Val Gly Asp Arg Val Asn Ile Thr Cys Lys

Ala Ser Gln Asn Val Gly Ser Asn Val Ala Trp Leu Gln Gln Lys Pro

Gly Gln Ser Pro Lys Thr Leu Ile Tyr Ser Ala Ser Tyr Arg Ser Gly

Arg Val Pro Asp Arg Phe Thr Gly Ser Gly Ser Gly Thr Asp Phe Ile

Leu Thr Ile Thr Thr Val Gln Ser Glu Asp Leu Ala Glu Tyr Phe Cys

Gln Gln Phe Asn Arg Ser Pro Leu Thr Phe Gly Ser Gly Thr Lys Leu

Glu Leu Lys Arg Ala Asp Ala Ala Pro Thr Val Ser Ile Phe Pro Pro

Ser Ser Lys Gly Glu Phe

## Claims

1. An anti-GM-CSF antibody for use in reducing the symptoms of rheumatoid arthritis in a patient undergoing treatment with methotrexate without inducing clinically significant neutropenia in which the absolute neutrophil count is less than 0.5x10⁹/l, wherein the anti-GM-CSF antibody is a GM-CSF antagonist, wherein the methotrexate is provided in an amount of 7.5 mg to 25 mg/week, and wherein the anti-GM-CSF antibody is provided in an amount of 0.1 to 25 mg/kg.

2. The anti-GM-CSF antibody for use according to claim 1, wherein the antibody
(a) is a polyclonal antibody;
(b) is a monoclonal antibody;
(c) is an antibody fragment that is a Fab, a Fab', a F(ab')₂, a scFv, or a dAB;
(d) is a high affinity antibody having a K_{D} for binding to human GM-CSF of less than 100 pM;
(e) is a neutralizing antibody;
(f) is a recombinant antibody;
(g) is a chimeric antibody;
(h) is a human antibody;
(i) comprises a human variable region;
(j) comprises a human light chain constant region;
(k) comprises a human heavy chain constant region;
(l) binds to the same epitope as chimeric 19/2;
(m) comprises the V_{H} and V_{L} regions of chimeric 19/2;
(n) comprises the V_{H} region and V_{L} region CDR1, CDR2, and CDR3 of chimeric 19/2; or
(o) comprises the V_{H} region CDR3 and V_{L} region CDR3 of chimeric 19/2.

3. The anti-GM-CSF antibody for use according to claim 1 or 2, wherein the antibody is a monoclonal antibody and, optionally, wherein the monoclonal antibody is a recombinant antibody.

4. The anti-GM-CSF antibody for use according to claim 3, wherein the antibody is humaneered.

5. The anti-GM-CSF antibody for use according to claim 3 or 4, wherein the antibody is a neutralizing monoclonal antibody that binds to the same epitope as chimeric 19/2.

6. The anti-GM-CSF antibody for use according to claim 5, wherein the antibody has a K_{D} ranging from about 5 pM to about 50 pM.

7. The anti-GM-CSF antibody for use according to claim 6, wherein the antibody has a K_{D} from about 10 pM to about 30 pM.

8. The anti-GM-CSF antibody for use according to claim 1, wherein the antibody is a neutralizing recombinant monoclonal antibody that binds to the same epitope as chimeric 19/2 and has a K_{D} of about 10 pM to about 30 pM.

9. The anti-GM-CSF antibody for use according to claim 1, wherein the antibody is an antibody fragment that is a Fab, Fab', F(ab')₂, scFv or dAB and is conjugated to polyethylene glycol.

10. The anti-GM-CSF antibody for use according to claim 1, wherein the antibody comprises a gamma region human heavy chain constant region, optionally a gamma 1 region.

11. The anti-GM-CSF antibody for use according to claim 1, wherein the antibody comprises the V_{H} and the V_{L} regions of chimeric 19/2 and a human heavy chain constant region.

12. The anti-GM-CSF antibody for use according to claim 11, wherein the human heavy chain constant region is a gamma region, optionally a gamma 1 region.

13. The anti-GM-CSF antibody for use according to claim 1, wherein the anti-GM-CSF antibody comprises a humaneered Fab' with the binding specificity of chimeric 19/2 and has a K_{D} ranging from about 5 to about 50 pM.

14. The anti-GM-CSF antibody for use according to any one of the preceding claims, wherein the antibody is administered subcutaneously or intravenously.

15. The anti-GM-CSF antibody for use according to any one of the preceding claims, wherein the antibody is provided in an amount of 0.2 to 10 mg/kg.

16. Use of an anti-GM-CSF antibody for the preparation of a medicament for reducing the symptoms of rheumatoid arthritis in a patient undergoing treatment with methotrexate without inducing clinically significant neutropenia in which the absolute neutrophil count is less than 0.5x10⁹/l, wherein the anti-GM-CSF antibody is an antagonist, wherein the anti-GM-CSF antibody is provided in an amount of 0.1 to 25 mg/kg, and wherein the methotrexate is provided in an amount of 7.5 mg to 25 mg/week.

## Patentansprüche

1. Anti-GM-CSF-Antikörper zur Verwendung zur Verringerung der Symptome der rheumatoiden Arthritis bei einem Patienten, der sich einer Behandlung mit Methotrexat unterzieht, ohne klinisch signifikante Neutropenie zu induzieren, bei dem die absolute Neutrophilenzahl weniger als 0,5 x 10⁹/l beträgt, wobei der Anti-GM-CSF-Antikörper ein GM-CSF-Antagonist ist, wobei das Methotrexat in einer Menge von 7,5 mg bis 25 mg/Woche verabreicht wird, und wobei der Anti-GM-CSF-Antikörper in einer Menge von 0,1 bis 25 mg/kg verabreicht wird.

2. Anti-GM-CSF-Antikörper zur Verwendung nach Anspruch 1, wobei der Antikörper
(a) ein polyklonaler Antikörper ist;
(b) ein monoklonaler Antikörper ist;
(c) ein Antikörperfragment, das ein Fab, ein Fab', ein F(ab')₂, ein scFv oder ein dAB ist, ist;
(d) ein hochaffiner Antikörper mit einem K_{D}-Wert zur Bindung an menschlichen GM-CSF von weniger als 100 pM ist;
(e) ein neutralisierender Antikörper ist;
(f) ein rekombinanter Antikörper ist;
(g) ein chimärer Antikörper ist;
(h) ein humaner Antikörper ist;
(i) eine humane variable Region umfasst;
(j) die konstante Region einer humanen leichten Kette umfasst;
(k) die konstante Region einer humanen schweren Kette umfasst;
(l) an das gleiche Epitop wie das chimäre 19/2 bindet;
(m) die V_{H}- und V_{L}-Regionen des chimären 19/2 umfasst;
(n) die V_{H}-Region und die V_{L}-Region CDR1, CDR2 und CDR3 des chimären 19/2 umfasst, oder
(o) die V_{H}-Region CDR3 und V_{L}-Region CDR3 des chimären 19/2 umfasst.

3. Anti-GM-CSF-Antikörper zur Verwendung nach Anspruch 1 oder 2, wobei der Antikörper ein monoklonaler Antikörper ist und, gegebenenfalls, wobei der monoklonale Antikörper ein rekombinanter Antikörper ist.

4. Anti-GM-CSF-Antikörper zur Verwendung nach Anspruch 3, wobei der Antikörper humanisiert ist.

5. Anti-GM-CSF-Antikörper zur Verwendung nach Anspruch 3 oder 4, wobei der Antikörper ein neutralisierender monoklonaler Antikörper ist, der das gleiche Epitop wie das chimäre 19/2 bindet.

6. Anti-GM-CSF-Antikörper zur Verwendung nach Anspruch 5, wobei der Antikörper einen K_{D}-Wert im Bereich von etwa 5 pM bis etwa 50 pM besitzt.

7. Anti-GM-CSF-Antikörper zur Verwendung nach Anspruch 6, wobei der Antikörper einen K_{D}-Wert von etwa 10 pM bis etwa 30 pM besitzt.

8. Anti-GM-CSF-Antikörper zur Verwendung nach Anspruch 1, wobei der Antikörper ein neutralisierender rekombinanter monoklonaler Antikörper ist, der an das gleiche Epitop wie das chimäre 19/2 bindet und einen K_{D}-Wert von etwa 10 pM bis etwa 30 pM besitzt.

9. Anti-GM-CSF-Antikörper zur Verwendung nach Anspruch 1, wobei der Antikörper ein Antikörperfragment ist, das ein Fab, Fab', F(ab')₂, scFv oder dAB ist, und an Polyethylenglykol konjugiert ist.

10. Anti-GM-CSF-Antikörper zur Verwendung nach Anspruch 1, wobei der Antikörper eine Gammaregion der konstanten Region der menschlichen schweren Kette, gegebenenfalls eine Gamma-1-Region, umfasst.

11. Anti-GM-CSF-Antikörper zur Verwendung nach Anspruch 1, wobei der Antikörper die V_{H}- und die V_{L}-Regionen des chimären 19/2 und eine konstante Region der menschlichen schweren Kette umfasst.

12. Anti-GM-CSF-Antikörper zur Verwendung nach Anspruch 11, wobei die konstante Region der menschlichen schweren Kette eine Gammaregion, gegebenenfalls eine Gamma-1-Region, ist.

13. Anti-GM-CSF-Antikörper zur Verwendung nach Anspruch 1, wobei der Anti-GM-CSF-Antikörper ein humanisiertes Fab' mit der Bindungsspezifität des chimären 19/2 umfasst, und einen K_{D}-Wert im Bereich von etwa 5 bis etwa 50 pM besitzt.

14. Anti-GM-CSF-Antikörper zur Verwendung nach einem der vorstehenden Ansprüche, wobei der Antikörper subkutan oder intravenös verabreicht wird.

15. Anti-GM-CSF-Antikörper zur Verwendung nach einem der vorstehenden Ansprüche, wobei der Antikörper in einer Menge von 0,2 bis 10 mg/kg verabreicht wird.

16. Verwendung eines Anti-GM-CSF-Antikörpers zur Herstellung eines Medikaments zur Verringerung der Symptome der rheumatoiden Arthritis bei einem Patienten, der sich einer Behandlung mit Methotrexat unterzieht, ohne klinisch signifikante Neutropenie zu induzieren, bei dem die absolute Neutrophilenzahl weniger als 0,5 x 10⁹/l beträgt, wobei der Anti-GM-CSF-Antikörper ein GM-CSF-Antagonist ist, wobei der Anti-GM-CSF-Antikörper in einer Menge von 0,1 mg bis 25 mg/kg verabreicht wird, und wobei das Methotrexat in einer Menge von 7,5 bis 25 mg/Woche verabreicht wird.

## Revendications

1. Anticorps anti-GM-CSF pour utilisation pour réduire les symptômes d'une polyarthrite rhumatoïde, chez un patient traité avec du méthotrexate, sans provoquer une neutropénie significative sur le plan clinique où le nombre absolu de neutrophiles deviendrait inférieur à 0,5.10⁹ par litre, lequel anticorps anti-GM-CSF est un antagoniste du GM-CSF, et dans laquelle utilisation le méthotrexate sera donné en une quantité de 7,5 mg à 25 mg par semaine et l'anticorps anti-GM-CSF sera donné en une quantité de 0,1 à 25 mg/kg.

2. Anticorps anti-GM-CSF pour utilisation conforme à la revendication 1, lequel anticorps
a) est un anticorps polyclonal ;
b) est un anticorps monoclonal ;
c) est un fragment d'anticorps qui est un fragment Fab, Fab', F(ab')₂, scFv ou dAB ;
d) est un anticorps de haute affinité, qui présente pour la liaison au GM-CSF humain une constante K_{D} inférieure à 100 pM ;
e) est un anticorps neutralisant ;
f) est un anticorps recombinant ;
g) est un anticorps chimérique ;
h) est un anticorps humain ;
i) comprend une région variable humaine ;
j) comprend une région constante de chaîne légère humaine ;
k) comprend une région constante de chaîne lourde humaine ;
l) se lie au même épitope que l'anticorps chimérique 19/2 ;
m) comprend les régions V_{H} et V_{L} de l'anticorps chimérique 19/2 ;
n) comprend les régions CDR1, CDR2 et CDR3 des régions V_{H} et V_{L} de l'anticorps chimérique 19/2 ;
o) ou comprend la région CDR3 de la région V_{H} et la région CDR3 de la région V_{L} de l'anticorps chimérique 19/2.

3. Anticorps anti-GM-CSF pour utilisation conforme à la revendication 1 ou 2, lequel anticorps est un anticorps monoclonal, et lequel anticorps monoclonal, en option, est un anticorps recombinant.

4. Anticorps anti-GM-CSF pour utilisation conforme à la revendication 3, lequel anticorps est un anticorps humain modifié.

5. Anticorps anti-GM-CSF pour utilisation conforme à la revendication 3 ou 4, lequel anticorps est un anticorps monoclonal neutralisant qui se lie au même épitope que l'anticorps chimérique 19/2.

6. Anticorps anti-GM-CSF pour utilisation conforme à la revendication 5, lequel anticorps présente une constante K_{D} valant d'environ 5 pM à environ 50 pM.

7. Anticorps anti-GM-CSF pour utilisation conforme à la revendication 6, lequel anticorps présente une constante K_{D} valant d'environ 10 pM à environ 30 pM.

8. Anticorps anti-GM-CSF pour utilisation conforme à la revendication 1, lequel anticorps est un anticorps monoclonal recombinant neutralisant qui se lie au même épitope que l'anticorps chimérique 19/2 et présente une constante K_{D} valant d'environ 10 pM à environ 30 pM.

9. Anticorps anti-GM-CSF pour utilisation conforme à la revendication 1, lequel anticorps est un fragment d'anticorps qui est un fragment Fab, Fab', F(ab')₂, scFv ou dAB, et est conjugué à du poly-éthylèneglycol

10. Anticorps anti-GM-CSF pour utilisation conforme à la revendication 1, lequel anticorps comprend une région constante de chaîne lourde humaine gamma, en option une région gamma 1.

11. Anticorps anti-GM-CSF pour utilisation conforme à la revendication 1, lequel anticorps comprend les régions V_{H} et V_{L} de l'anticorps chimérique 19/2 et une région constante de chaîne lourde humaine.

12. Anticorps anti-GM-CSF pour utilisation conforme à la revendication 11, dans lequel la région constante de chaîne lourde humaine est une région gamma, en option une région gamma 1.

13. Anticorps anti-GM-CSF pour utilisation conforme à la revendication 1, lequel anticorps anti-GM-CSF comprend un fragment Fab' humain modifié, doté de la spécificité de liaison de l'anticorps chimérique 19/2, et présente une constante K_{D} valant d'environ 5 à environ 50 pM.

14. Anticorps anti-GM-CSF pour utilisation conforme à l'une des revendications précédentes, dans laquelle l'anticorps sera administré par voie sous-cutanée ou intraveineuse.

15. Anticorps anti-GM-CSF pour utilisation conforme à l'une des revendications précédentes, dans laquelle l'anticorps sera donné en une quantité de 0,2 à 10 mg/kg.

16. Utilisation d'un anticorps anti-GM-CSF en vue de la préparation d'un médicament conçu pour réduire les symptômes d'une polyarthrite rhumatoïde, chez un patient traité avec du méthotrexate, sans provoquer une neutropénie significative sur le plan clinique où le nombre absolu de neutrophiles deviendrait inférieur à 0,5.10⁹ par litre, dans laquelle l'anticorps anti-GM-CSF est un antagoniste, l'anticorps anti-GM-CSF sera donné en une quantité de 0,1 à 25 mg/kg et le méthotrexate sera donné en une quantité de 7,5 mg à 25 mg par semaine.
